(19) Europäisches Patentamt
European Patent Office
Office européen des brevets

(11) Numéro de publication : **0 452 161 A1**

(12) **DEMANDE DE BREVET EUROPEEN**

(21) Numéro de dépôt : **91400320.7**

(22) Date de dépôt : **11.02.91**

(51) Int. Cl.$^5$ : **A61K 7/48,** A61K 7/06, A61K 7/16, C08G 69/10

(30) Priorité : **12.02.90 FR 9001618**

(43) Date de publication de la demande : **16.10.91 Bulletin 91/42**

(84) Etats contractants désignés : **AT BE CH DE DK ES FR GB GR IT LI LU NL SE**

(71) Demandeur : **SANOFI**
**40, Avenue George V**
**F-75008 Paris (FR)**

(72) Inventeur : **Barbier, Alain**
**280, avenue Miradou-Le Belvédère**
**F-34980 Saint Clément la Rivière (FR)**
Inventeur : **Millan, Joseph**
**106, rue des Cigales**
**F-34990 Juvignac (FR)**
Inventeur : **Sabadie, Michel**
**Rue de la Fontaine St Germain**
**F-27300 Bernay (FR)**

(74) Mandataire : **Gillard, Marie-Louise et al**
**Cabinet Beau de Loménie 55, Rue d'Amsterdam**
**F-75008 Paris (FR)**

(54) **Composition cosmétique contenant des copolymères d'aminoacides, utile comme agent hydratant.**

(57) L'invention a pour objet l'utilisation cosmétique d'un copolymère d'acides aminés de formule statistique :

$$-\left(A_m-\left(NH-\underset{\underset{COOH}{(CH_2)_a}}{CH}-CO\right)_n-B_p-\left(NH-\underset{\underset{COOH}{(CH_2)_b}}{CH}-CO\right)_q-\right)_z \qquad (I)$$

dans laquelle :

— a et b valent chacun indépendamment 1 ou 2 ;

— A et B sont semblables ou différents et représentent un acide aminé choisi parmi : Arginine, Sérine, Proline, Glycine, Histidine, Alanine, Lysine, Ornithine, Citrulline, Acide urocanique, Asparagine ou Tyrosine ;

— m, n, p, q sont des nombres entiers tels que le rapport $\frac{n+q}{m+p}$ est compris entre 0,5 et 4

— z est un nombre entier tel que le poids moléculaire moyen est compris entre 1000 et 100000 et/ou un de ses sels.

EP 0 452 161 A1

# COMPOSITION COSMETIQUE CONTENANT DES COPOLYMERES D'AMINOACIDES, UTILE COMME AGENT HYDRATANT

La présente invention a pour objet l'utilisation de certains copolymères d'aminoacides comme agents hydratants et les compositions cosmétiques contenant lesdits copolymères d'aminoacides.

Dans la description de la présente invention et dans les revendications qui vont suivre, les abréviations utilisées pour les acides aminés sont celles recommandées par l'IUPAC.

Il est connu par les demandes de brevets français publiées sous les n° 2 609 393 et 2 540 381 d'utiliser des composés peptidiques d'origine naturelle pour préparer des compositions pharmaceutiques, dermatologiques ou cosmétiques. Il est également connu par la demande de brevet japonais 59-209635 d'utiliser l'acide polyglutamique comme agent hydratant dans des shampooings, lotions ou crèmes.

Cependant, l'action de l'acide polyglutamique ou celle d'autres mélanges de polymères d'aminoacides testés jusqu'à ce jour est très fugace, ce qui diminue l'intérêt de leur utilisation dans des compositions cosmétiques destinées à l'hydratation de la peau.

On a maintenant trouvé que l'on peut utiliser certains copolymères d'aminoacides pour leurs propriétés hydratantes durables.

La présente invention a pour objet des compositions cosmétiques contenant un ou plusieurs copolymères d'acides aminés, lesdits copolymères ayant des propriétés hydratantes importantes et rémanentes. De plus, lesdits copolymères sont parfaitement bien tolérés par la peau.

Selon la présente invention, on appelle polymère ou copolymère d'acides aminés un composé comprenant un enchaînement d'acides aminés dont le poids moléculaire moyen (PM) est compris entre 1 000 et 100 000.

Les copolymères d'acides aminés utilisés comme ingrédients actifs dans les compositions cosmétiques selon la présente invention sont préparés par copolymérisation de 2 ou 3 acides aminés parmi lesquels se trouve au moins un acide aminé portant une fonction acide sur sa chaîne latérale, à savoir l'acide glutamique ou l'acide aspartique, le ou les 2 autres acides aminés étant choisis parmi les acides aminés constitutifs de la filaggrine et/ou du facteur naturel d'hydratation (NMF : natural moisturizing factor). Les acides aminés résultant de la décomposition de la filaggrine sont décrits par MANSBRIGE dans Arch. Dermatol. Rev., 1987, 279, 465-469. La composition du NMF est décrite dans Labo. Pharma. Problèmes et Techniques, 1973, 273, 82.

Ainsi, selon la présente invention, l'acide glutamique et/ou l'acide aspartique forment un copolymère avec un autre acide aminé ou éventuellement 2 autres acides aminés, lesdits acides aminés étant choisis parmi les suivants : Arginine, Sérine, Proline, Glycine, Histidine, Alanine, Lysine, Ornithine, Citrulline, Acide urocanique, Asparagine ou Tyrosine.

Le copolymère contient dans sa formule, de façon statistique, une proportion d'acide glutamique ou d'acide aspartique ou bien d'acide glutamique et d'acide aspartique ensemble, comprise entre 0,5 et 4 molaire vis-à-vis du ou des deux autres acides aminés avec lesquels ils forment le copolymère.

Ledit copolymère dont l'utilisation comme agent hydratant est un objet de la présente invention peut être représenté par la formule générale statistique suivante :

$$\left( -A_m-\left( NH-\underset{\underset{COOH}{|}}{\underset{(CH_2)_a}{\underset{|}{CH}}}-CO \right)_n -B_p-\left( NH-\underset{\underset{COOH}{|}}{\underset{(CH_2)_b}{\underset{|}{CH}}}-CO \right)_q - \right)_z \qquad (I)$$

dans laquelle :

– a et b valent chacun indépendamment 1 ou 2 ;

– A et B sont semblables ou différents et représentent un acide aminé choisi parmi : Arginine, Sérine, Proline, Glycine, Histidine, Alanine, Lysine, Ornithine, Citrulline, Acide urocanique, Asparagine ou Tyrosine ;

– m, n, p, q sont des nombres entiers tels que le rapport $\frac{n+q}{m+p}$ est compris entre 0,5 et 4 ;

– z est un nombre entier tel que le poids moléculaire moyen est compris entre 1 000 et 100 000.

Les sels dudit copolymère avec les bases minérales et organiques compatibles avec une utilisation cosmétologique font également partie de l'invention.

On notera que la formule (I) est une formule statistique, c'est-à-dire que les enchaînements des acides

aminés indiqués sont arbitraires et peuvent se présenter sous toutes autres combinaisons.

Les compositions cosmétiques selon la présente invention sont caractérisées en ce qu'elles contiennent de 0,1 à 10 % en poids d'un copolymère de formule (I) ou d'un mélange d'au moins 2 copolymères de formule (I) dans un excipient cosmétique.

Préférentiellement, une composition cosmétique selon l'invention contient de 0,5 à 5 % en poids d'un copolymère ou d'un mélange de 2 copolymères (I) dans un excipient cosmétique.

Chacun des acides aminés choisis parmi : Arginine, Sérine, Proline, Glycine, Histidine, Alanine, Lysine, Ornithine, Citrulline, Acide urocanique, Asparagine ou Tyrosine peut former un copolymère (I) avec l'acide glutamique et/ou l'acide aspartique et conduire ainsi à un composé ayant des propriétés hydratantes pour la peau ; lesdites propriétés se manifestant de façon durable.

Lorsque l'on utilise un mélange de 2 copolymères (I), l'acide glutamique et/ou l'acide aspartique est copolymérisé avec des acides aminés A et éventuellement B différents.

La rapidité avec laquelle le pouvoir hydratant du copolymère (I) se manifeste, ainsi que la rémanence plus ou moins longue de ce pouvoir hydratant peuvent varier avec l'acide aminé choisi.

Ainsi, en choisissant convenablement un mélange de 2 copolymères (I), on peut préparer une composition cosmétique selon l'invention ayant un pouvoir hydratant à la fois précoce et durable par exemple dès 30 min après l'application et pendant une période de temps au moins égale à 24 h.

Ainsi, de façon préférentielle, les compositions cosmétiques selon l'invention contiennent un mélange de 2 copolymères (I).

Le copolymère (I) entrant en tant qu'ingrédient actif dans une composition cosmétique selon l'invention contient de façon préférentielle de l'acide glutamique dans une proportion molaire de 0,5 à 4 ; ceci se traduit par les valeurs suivantes des coefficients de la formule générale statistique (I) :

$a = b = 2$ et $\frac{n + q}{m + p}$ compris entre 0,5 et 4.

Plus particulièrement, l'acide glutamique est en rapport molaire avec un seul autre acide aminé, dans ce cas, les valeurs des coefficients de la formule statistique du copolymère (I) sont :

$A = B, a = b = 2$ et $\frac{n + q}{m + p} = 1$

Enfin, de façon tout à fait préférentielle, la composition cosmétique selon l'invention contient un copolymère choisi parmi Lys-Glu (1/1) et Pro-Glu (1/1) ou, préférentiellement, un mélange de ces 2 copolymères.

Les copolymères d'acides aminés (I) sont connus ou préparés selon des méthodes connues.

Les acides aminés, activés ou non,et protégés le cas échéant sur leur fonction latérale,se polymérisent en présence d'un initiateur basique, par exemple une amine secondaire ou une amine tertiaire, telle que la triéthylamine. La réaction est effectuée dans un solvant dans lequel le monomère et le polymère sont solubles, par exemple le chlorure de méthylène, le diméthylformamide, le diméthylsulfoxyde ou le dioxanne (J. Am. Chem. Soc., 1956, 78, 941-946). La polymérisation peut être conduite à partir du N-carboxyanhydride de l'aminoacide, préparé selon H. Leuchs (Ber. Dtsch. Chem. Ges., 1906, 39, 857). Cette méthode peut aussi être appliquée pour préparer un copolymère de 2 ou 3 acides aminés différents.

On peut également effectuer la réaction de polymérisation par l'action d'un initiateur sur les acides aminés sous forme d'esters activés.

Les copolymères ainsi obtenus présentent des répartitions statistiques des acides aminés et un poids moléculaire moyen compris entre 1 000 et 100 000.

Si l'on cherche à obtenir des polymères dans lesquels la répartition des acides aminés soit régulière dans une séquence, on peut préparer, par des méthodes classiques de synthèse peptidique, les segments polypeptidiques correspondant à l'enchaînement souhaité, puis préparer les esters actifs correspondants et effectuer ensuite la polymérisation de ces polypeptides en présence d'un initiateur basique selon le procédé précédemment décrit. Le polymère ainsi obtenu présente une répartition régulière des acides aminés ; son poids moléculaire moyen correspond au nombre statistique de polypeptides qui ont polymérisé.

Pour obtenir un polymère (I) régulier et de poids moléculaire rigoureusement défini, on peut effectuer une synthèse en phase solide à partir des acides aminés nécessaires à la préparation du copolymère.

Aux différentes étapes de la préparation, les polymères d'acides aminés sont purifiés par les techniques de chromatographie de perméation sur gel et d'ultrafiltration qui permettent la séparation des molécules selon leur poids moléculaire. La chromatographie de perméation sur gel est effectuée d'après les techniques décrites dans Sci. Report of Toyo Soda, 1985, 29, (1) 37-54.

L'ultrafiltration est réalisée sur des cellules Amicon® commercialisées par Amicon.

La chromatographie de perméation sur gel, associée à la réfractométrie, sert également à la détermination

du poids moléculaire moyen des polymères, selon la technique décrite par D. Lecacheux et R. Panaras dans Carbohydrate Polymers, 1985, 5, 423-440. Dans ce cas, on effectue la chromatographie, puis l'analyse de la diffusion de la lumière des copolymères par rapport à des substances de référence ayant un poids moléculaire connu, telles que des polyoxyéthylènes, qui servent à établir une courbe d'étalonnage.

Le poids moléculaire moyen (PM) est associé à un indice de polydispersité qui exprime la répartition statistique des polymères de différents poids moléculaires autour du poids moléculaire moyen.

Les compositions cosmétiques selon la présente invention contiennent, à titre d'ingrédient actif, de 0,1 à 10 %, de préférence de 0,5 à 5 % en poids d'un copolymère d'acides aminés (I) ou d'un mélange d'au moins deux copolymères (I) tels que définis selon la présente invention.

Le ou les copolymères (I) sont mélangés avec des excipients cosmétiques pour préparer des crèmes, des lotions, des émulsions ou des solutions.

Pour préparer des compositions cosmétiques, les composants sont mélangés aux excipients généralement employés dans la technique cosmétique tels que, par exemple, les matières grasses d'origine animale ou végétale, les huiles végétales, les acides gras, les alcools, les glycols polyalkyléniques, les cires, les hydrocarbures, les polyesters et peuvent être associés à l'eau et à des agents gélifiants lorsqu'ils sont compatibles. Dans ces préparations, on peut ajouter d'autres ingrédients compatibles avec les composants comme des agents conservateurs, tels que les esters de l'acide 4-hydroxybenzoïque, des antioxydants, comme le butyl-hydroxytoluène ou les dérivés de la vitamine E, ou des parfums.

Les acides gras, utilisés comme adjuvants dans les compositions cosmétiques de la présente invention, peuvent être saturés ou insaturés, contenir de 1 à 22 atomes de carbone, être non substitués ou substitués avec un groupe hydroxyle, sous forme libre ou sous forme d'un de leurs sels alcalins.

La forme des compositions cosmétiques selon l'invention peut notamment être une crème dans laquelle les composants sont associés aux excipients couramment utilisés dans la cosmétologie, et compatibles avec lesdits composants, tels que la lanoline, ou ses dérivés.

Les compositions cosmétiques de l'invention peuvent être également présentées sous forme de gel dans les excipients appropriés, tels que les esters de cellulose, les polymères acryliques, les polymères méthacryliques, en particulier le polyglycéryl méthacrylate, ou d'autres agents gélifiants.

Les compositions cosmétiques suivant l'invention peuvent aussi prendre la forme de lotion, de solution ou de microémulsion dans lesquelles les composants sont dissous ou microdispersés.

La forme des compositions cosmétiques suivant l'invention peut donc être une microdispersion de composants dans un liquide contenant de l'eau, ainsi qu'un ou plusieurs agents tensioactifs. Ces dispersions présentent les caractères des microémulsions et ont pratiquement l'aspect de solutions vraies.

Ces microémulsions jouissent d'une bonne stabilité et peuvent être conservées, pendant le temps nécessaire pour l'utilisation, à des températures comprises entre 0 et 60°C sans qu'il y ait sédimentation des constituants ou séparation de phases de façon irréversible. Si nécessaire, les compositions peuvent également être préparées extemporanément.

Les tensioactifs de la composition sont choisis parmi les agents de surface utilisables en cosmétologie. On peut indiquer à titre d'exemples non limitatifs : les esters de sorbitol et leurs dérivés polyoxyéthylénés, les huiles de ricin (hydrogénées ou non) polyoxyéthylénées, les copolymères séquencés oxyde d'éthylène/oxyde de propylène, les alcools gras et les stérols polyoxyéthylénés, le laurylsulfate de sodium, le dioctylsulfosuccinate de sodium, les lécithines d'oeuf ou de soja, les huiles de silicones polyoxyéthylénées.

Selon la présente invention, on peut également incorporer des copolymères (I) dans des biovecteurs, tels que les liposomes ou toute autre forme adaptée à une utilisation cosmétique et permettant d'assurer une distribution tissulaire sélective optimale.

Les compositions cosmétiques de la présente invention sont très bien tolérées ; elles ne présentent aucune phototoxicité et leur application sur la peau pour des périodes de temps prolongées n'implique aucun effet systémique.

Les compositions cosmétiques de la présente invention sont plus particulièrement destinées à :

– améliorer l'état d'hydratation de la peau, en traitement curatif ou préventif, sur des peaux desséchées, vieillies ou brûlées par le soleil ;

– favoriser la réparation tissulaire de peaux vieillies ou endommagées par des facteurs exogènes.

Le pouvoir hydratant des copolymères (I) a été étudié chez l'animal en appliquant des méthodes proches de celles décrites par D. Wilson et al. dans Int. J. Cosmet. Sci., 1988, 10, 201-211.

On utilise des cobayes "sans poils" de souche HAIRLESS. On opère dans des conditions de température (22°C ± 2) et d'humidité relative stables et on évite les mouvements d'air.

Pour la préparation des animaux, deux zones ont été délimitées sur le dos de chaque cobaye :

– une zone témoin, côté gauche

– une zone traitée, côté droit.

Tous les cobayes reçoivent 0,2 ml d'eau distillée sur le côté gauche et 0,2 ml d'une solution aqueuse à 5 % du copolymère à tester sur le côté droit.

Pour les mesures, on utilise, d'une part, le cornéomètre CM 420 et, d'autre part, l'évaporimètre EP1.

Le cornéomètre CM 420 est fabriqué par la firme Schwarzhaupt et permet la mesure du pouvoir hydratant des cosmétiques. Cet appareil est constitué d'une console et d'une sonde qui enregistre la conductance, valeur proportionnelle à la teneur en eau. La mesure est obtenue par application de la sonde à l'endroit désiré et on observe l'affichage d'un nombre sur le cadran de la console.

L'évaporimètre EP1 est commercialisé par la société Servo Med et permet la mesure de perte d'eau transépidermique.

Le taux d'évaporation de l'eau à la surface de la peau est mesuré grâce à une méthode basée sur la mesure du gradient de pression de vapeur d'eau dans la couche d'air immédiatement adjacente à la peau. L'appareil possède une sonde ayant des détecteurs d'humidité et de température. La mesure de la perte d'eau transépidermique est réalisée en appliquant la sonde, pendant 30 s, à l'endroit désiré. La perte d'eau transépidermique ne mesure pas le contenu d'eau de Stratum Corneum mais la vitesse à laquelle l'eau diffuse vers la surface. Cette mesure donne une évaluation de la qualité de la fonction barrière naturelle de la peau.

Pour évaluer l'effet hydratant du produit, on a traité les animaux pendant 2 semaines.

Mesures et traitement

Pour chaque animal, trois mesures ont été réalisées, chacun des jours correspondant à un jour de traitement.

La première mesure $T_0$ est effectuée, chaque jour, avant le traitement de l'animal. Cette mesure, comme les suivantes, a été faite sur la zone traitée et sur la zone témoin. Cette détermination à $J_0$ correspond à la valeur de base de la zone considérée et permet d'apprécier les jours suivants l'effet rémanent à 24 h.

La deuxième mesure $T_1$ est réalisée 30 min après le traitement de façon à pouvoir apprécier l'effet du produit juste après son application sans que l'effet de l'eau soit encore sensible.

La troisième mesure $T_2$ est effectuée 4 h après le traitement de manière à déterminer si le produit possède une action prolongée dans le temps.

Les résultats obtenus montrent que les composés selon l'invention entraînent une augmentation nette et durable de l'hydratation cutanée, mesurée par cornéométrie, qui s'accompagne dans certains cas d'une diminution des pertes d'eau transépidermiques (p.i.e.), mesurées par évaporimétrie.

En conclusion, ces produits présentent une activité hydratante nette et rémanente sur 24 h.

Dans les exemples et dans les revendications qui vont suivre, les abréviations suivantes sont utilisées :

TFA : acide trifluoroacétique.
HBr : acide bromhydrique.
OBzl : ester benzylique.
ONp : ester p-nitrophénylique.
Z : benzyloxycarbonyle.
DMF : diméthylformamide.
DCM : dichlorométhane.
OHBT : hydroxybenzotriazole.
DCC : dicyclohexylcarbodiimide.
DMSO : diméthylsulfoxyde.
DCU : dicyclohexylurée.

Dans un souci de simplification, certains constituants des compositions cosmétiques ont été désignés par leur dénomination commerciale ou par un sigle dont voici les significations :

Carbopol 940 : carboxypolyméthylène, commercialisé par GOODRICH
EDTA : acide éthylènediaminotétracétique
Filtre UVB : 4-méthoxycinnamate d'éthyle-2-hexyle (marque PARSOL MCX)

Les exemples suivants, non limitatifs, illustrent la préparation des copolymères (I) et des compositions cosmétiques selon l'invention.

EXEMPLE 1

Copolymère Glu-Lys (1/1) : SR 46512.

A) Copolymère Glu (OBzl)-Lys (Z) (1/1).

On dissout dans 200 ml de DMF 32 g de TFA, H-Lys (Z)-ONp et 29,32 g de TFA, H-Glu (OBzl)-ONp. On ajoute 200 ml de DCM. On place sous agitation, on refroidit le milieu à 5°C et on ajoute en une fois 17,2 ml de triéthylamine puis on laisse au repos à température ambiante pendant 4 jours. On dilue le milieu réactionnel par 100 ml de DMF et on ajoute 1,72 ml de triéthylamine supplémentaire puis on laisse au repos pendant 24 h. Le mélange réactionnel est ensuite versé dans 2 l d'éther éthylique fortement agité.

On essore le précipité obtenu et on chasse le solvant sous vide jusqu'à obtention d'un poids constant de solide jaune clair, M = 25,92 g.

Le produit est utilisé tel quel à l'étape suivante.

B) Copolymère Glu-Lys (1/1), HBr.

15,8 g de copolymère obtenu à l'étape précédente sont dissous dans 150 ml de TFA. On fait passer dans le milieu un courant d'acide bromhydrique gazeux pendant 30 min puis laisse agiter à température ambiante pendant 1 h. On verse le milieu réactionnel dans 2 l d'éther éthylique à 0°C. Après 1 h au repos à 0°C, le précipité formé est essoré, lavé abondamment à l'éther et séché sous vide en présence de potasse. On obtient 12,90 g de bromhydrate du copolymère Glu-Lys (1/1).

C) Copolymère Glu-Lys (1/1).

Le copolymère obtenu à l'étape précédente est dissous dans 500 ml d'eau et on mesure un pH-1,2. On ajoute 200 ml de Résine I.R. 45 (Amberlite)®. Le pH du milieu est pH = 7. On agite pendant 15 min et on filtre. Le filtrat est concentré sous vide à une température comprise entre 30 et 35°C, puis lyophilisé. On obtient 7,35 g d'un produit pulvérulent crème.

L'analyse d'aminoacides est conforme à une lysine pour un acide glutamique. Le poids moléculaire approché déterminé par la technique de chromatographie par perméation sur gel est PM = 1440. L'analyse du spectre de RMN à 250 MHz confirme l'absence de signaux dus aux noyaux aromatiques.

<u>EXEMPLE 2</u>

Copolymère Pro-Glu (1/1) : SR 44478

A) Copolymère Pro-Glu (OBzl) (1/1).

On dissout dans 160 ml de DMF 25,5 g de TFA, H-Pro-ONp et 34,39 g de TFA, H-Glu (OBzl)-ONp. On ajoute 160 ml de DCM. On place sous agitation, on refroidit le milieu à 5°C et on ajoute en une fois 20,18 ml de triéthylamine puis on laisse au repos, à température ambiante, pendant 5 jours. On ajoute 2 ml de triéthylamine supplémentaire puis on laisse au repos pendant 48 h. Le mélange réactionnel est ensuite concentré à sec sous vide. Le résidu est dissous dans 500 ml de DCM et la solution est lavée 3 fois par 500 ml d'une solution aqueuse de carbonate acide de sodium à 5 %, puis par 500 ml d'eau. La phase organique est séchée sur sulfate de magnésium et concentrée sous vide à 30°C jusqu'à obtention d'un poids constant de mousse solide jaune : 22,74 g. Le produit est utilisé tel quel à l'étape suivante.

B) Copolymère Pro-Glu (1/1).

22,74 g de copolymère obtenu à l'étape précédente sont dissous dans 300 ml de TFA. On fait passer dans le milieu un courant d'acide bromhydrique gazeux pendant 60 min puis agite à température ambiante pendant 4 h. On verse le mélange réactionnel dans 2 l d'éther éthylique à 0°C. Après 1 h au repos à 0°C, le précipité formé est essoré, lavé 3 fois par 500 ml d'éther éthylique et séché sous vide dans un dessiccateur, en présence de potasse : 16,47 g. On dissout le produit dans la quantité nécessaire de soude normale jusqu'a dissolution complète. On purifie à l'aide d'un boyau à dialyse contre de l'eau distillée pendant 24 h à température ambiante. Le produit restant dans le boyau à dialyse est concentré sous vide à température comprise entre 30 et 35°C, puis lyophilisé. On obtient 10,36 g d'un produit pulvérulent jaune clair. Ce produit est purifié à nouveau par une chromatographie de perméation sur gel sur une colonne de Biogel® P 10 en éluant par une solution aqueuse d'acide acétique à 2,5 % en volume. On obtient 9,80 g d'un produit pulvérulent crème. L'analyse d'aminoacides est conforme à une proline pour un acide glutamique.

Le poids moléculaire moyen approché déterminé par la technique de chromatographie par perméation sur

gel est PM = 7600. L'analyse du spectre de RMN enregistré à 250 MHz confirme l'absence de signaux dus aux noyaux aromatiques.

EXEMPLE 3

Copolymère Pro-Glu (1/1) : SR 44478.

A) Copolymère Pro-Glu (OBzl) (1/1).

5 g de H-Pro-OH et 10,3 g de H-Glu(OBzl)-OH sont mis en suspension dans 200 ml de DMSO. On ajoute 6,62 ml de TFA puis, quand tout est dissous, 11,92 ml de triéthylamine, 17,71 g de DCC et 11,62 g de OHBT. On laisse sous agitation à température ambiante une nuit, on rajoute 1,77 g de DCC et on laisse à nouveau sous agitation pendant 24 h. On filtre la DCU formée et on verse le filtrat sur environ 2 l d'eau avec de la glace. On extrait 2 fois par 1 l de DCM, sèche la solution organique sur sulfate de magnésium et concentre sous vide à 50°C jusqu'à obtention d'un poids constant d'huile. Cette huile est dissoute dans 100 ml de DCM et on laisse refroidir à 0°C. Le deuxième précipité de DCU qui se forme alors est filtré et le filtrat est versé dans 1 l d'hexane fortement agité. Le précipité formé est essoré, lavé à l'hexane et séché.

On obtient 8,6 g d'un produit pulvérulent de couleur crème.

B) Copolymère Pro-Glu (1/1).

8,5 g de copolymère obtenu à l'étape précédente sont dissous dans 100 ml de TFA. On fait passer dans le milieu un courant d'acide bromhydrique gazeux pendant 30 min puis agite à température ambiante pendant 30 min. On verse le mélange réactionnel dans 1 l d'éther éthylique à 0°C. Après 1 h au repos à 0°C, le précipité formé est essoré, lavé 3 fois par 100 ml d'éther éthylique. On dissout le produit dans 200 ml d'une solution aqueuse saturée de carbonate acide de sodium et dialyse la solution pendant 24 h contre de l'eau dans un dialyseur AMICON® à cartouches à fibres creuses (seuil de coupure : 2 000). On concentre la solution dialysée sous vide à 50°C jusqu'à obtention d'un poids constant de mousse solide marron clair : 6,6 g.

Ce produit est purifié à nouveau par une chromatographie de perméation sur gel sur une colonne PHARMACIA® K 50 garnie de FRACTOGEL® TSK HW 40 en éluant par de l'eau. On obtient deux fractions de produit pulvérulent :

1) 3,1 g d'un PM de 6 200 et indice de polydispersité = 1,5

2) 1 g d'un PM de 4 000 et indice de polydispersité = 1,5

mesurés par la technique de chromatographie par perméation de gel.

L'analyse d'aminoacides ainsi que la RMN à 250 MHz sont conformes à une proline pour un acide glutamique.

Dans les exemples de compositions cosmétiques, on désigne par COPOLYMERE un copolymère (I) ou un mélange de 2 copolymères (I).

EXEMPLE 4

GEL HYDRATANT

| | |
|---|---|
| COPOLYMERE | 3,00 g |
| Carbopol 940 | 0,20 g |
| Polyéthylèneglycol | 3,00 g |
| Conservateurs | 0,50 g |
| Butylèneglycol | 5,00 g |
| Laurate de sorbitan éthoxylé | 0,50 g |
| Parfum | 0,20 g |
| Triéthanolamine | 0,25 g |
| Eau déminéralisée | qsp 100 g |

EXEMPLE 5

BASE FLUIDE DE MAQUILLAGE

| | |
|---|---|
| COPOLYMERE | 1,00 g |
| Stérols de soja éthoxylés | 4,00 g |
| Stérols de soja | 0,50 g |
| Monostéarate de glycérol | 1,00 g |
| Huile végétale | 1,50 g |
| Palmitate d'éthyle hexyle | 4,00 g |
| Alcool cétylique | 0,50 g |
| Triglycérides capriques/capryliques | 1,50 g |
| Huile de silicone | 1,00 g |
| Huile minérale | 1,80 g |
| Alcools de lanoline | 0,20 g |
| Dipélargonate de propylèneglycol | 3,00 g |
| Lécithine | 1,00 g |
| Conservateurs | 0,50 g |
| Butylèneglycol | 5,00 g |
| Carbopol | 0,20 g |
| Triéthanolamine | 0,20 g |
| EDTA tétrasodique | 0,10 g |
| Antioxydant | 0,01 g |
| Parfum | 0,30 g |
| Eau déminéralisée | qsp 100 g |

## EXEMPLE 6

CREME DE JOUR PROTECTRICE

| | |
|---|---|
| COPOLYMERE | 3,00 g |
| Monostéarate de sorbitan éthoxylé | 2,60 g |
| Huile de silicone | 1,00 g |
| Alcool cétylique | 2,00 g |
| Huile minérale | 3,00 g |
| Alcool de lanoline | 1,00 g |
| Perhydrosqualène | 1,00 g |
| Monostéarate de sorbitan | 2,40 g |
| Palmitate de cétyle | 3,00 g |
| Palmitate d'isopropyle | 4,00 g |
| Filtre UVA - UVB | 2,00 g |
| EDTA tétrasodique | 0,10 g |
| Carbopol | 0,30 g |
| Triéthanolamine | 0,30 g |
| Antioxydant | 0,01 g |
| Conservateurs | 0,50 g |
| Parfum | 0,30 g |
| Butylèneglycol | 5,00 g |
| Eau déminéralisée | qsp 100 g |

## EXEMPLE 7

MICROEMULSIONS HYDRATANTES

| | |
|---|---|
| COPOLYMERE | 5,00 g |
| Hydroxystéarate de polyéthylèneglycol 600 | 1,00 g |
| Triglycérides (7-8 atomes de carbone) polyoxyéthylénés | 0,25 g |
| Polyéthylèneglycol-7 glycéryl cocoate | 0,20 g |
| Diméthicone copolyol | 0,25 g |
| Propylèneglycol | 12,50 g |
| Ethanol | 12,50 g |
| Carbopol | 0,40 g |
| Triéthanolamine | 0,40 g |
| Parfum | 0,30 g |
| Colorants | Q.S. |
| Conservateurs | 0,5 g |
| Eau déminéralisée | qsp 100 g |

EXEMPLE 8

FOND DE TEINT HYDRATANT

| | |
|---|---|
| COPOLYMERE | 2,00 g |
| EDTA tétrasodique | 0,10 g |
| Carboxyméthylcellulose | 0,20 g |
| Silicate d'aluminium magnésium | 1,00 g |
| Laurate de sorbitan éthoxylé | 1,00 g |
| Propylèneglycol | 5,00 g |
| Oxyde de titane | 2,00 g |
| Talc | 1,00 g |
| Pigments | 1,00 g |
| Triéthanolamine | 0,50 g |
| Conservateurs | 0,50 g |
| Alcool cétylique | 1,00 g |
| Alcool de lanoline | 3,00 g |
| Acide stéarique | 1,80 g |
| Monostéarate de propylèneglycol | 3,00 g |
| Palmitate d'isopropyle | 8,00 g |
| Huile végétale | 2,00 g |
| Antioxydant | 0,05 g |
| Parfum | 0,30 g |
| Eau déminéralisée | qsp 100 g |

EXEMPLE 9

CREME DE NUIT

| | |
|---|---|
| COPOLYMERE | 3,00 g |
| Alcool cétylique | 2,00 g |
| stéarine | 2,50 g |
| Monostéarate de glycérol | 5,00 g |
| Palmitate d'isopropyle | 5,00 g |
| Huile végétale | 3,00 g |
| Huile minérale | 2,00 g |
| Perhydrosqualène | 2,00 g |

| | |
|---|---|
| Huile de silicone | 1,00 g |
| Beurre de Karité | 2,00 g |
| Palmitate d'éthyle 2 hexyle | 5,00 g |
| Triéthanolamine | 5,50 g |
| Conservateurs | 0,50 g |
| Butylèneglycol | 5,00 g |
| EDTA tétrasodique | 0,30 g |
| Parfum | 0,31 g |
| Antioxydant | 0,10 g |
| Eau déminéralisée | qsp 100 g |

EXEMPLE 10

SERUM HYDRATANT

| | |
|---|---|
| COPOLYMERE | 5,00 g |
| Hydroxypropylméthylcellulose | 0,30 g |
| Propylèneglycol | 5,00 g |
| Glycérine | 5,00 g |
| Alcool oléique éthoxylé | 0,50 g |
| Parfum | 0,30 g |
| EDTA tétrasodique | 0,10 g |
| Conservateurs | 0,50 g |
| Albumine bovine | 5,00 g |
| Eau déminéralisée | qsp 100 g |

## Revendications

1. Utilisation d'un copolymère d'acides aminés de formule statistique :

$$-\left( A_m - \left\{ NH - \underset{\underset{COOH}{|}}{\underset{(CH_2)_a}{\underset{|}{CH}}} - CO \right\}_n - B_p - \left\{ NH - \underset{\underset{COOH}{|}}{\underset{(CH_2)_b}{\underset{|}{CH}}} - CO \right\}_q - \right)_z \quad (I)$$

dans laquelle

- a et b valent chacun indépendamment 1 ou 2 ;
- A et B sont semblables ou différents et représentent un acide aminé choisi parmi : Arginine, Sérine, Proline, Glycine, Histidine, Alanine, Lysine, Ornithine, Citrulline, Acide urocanique, Asparagine ou Tyrosine ;
- m, n, p, q sont des nombres entiers tels que le rapport $\frac{n + q}{m + p}$ est compris entre 0,5 et 4
- z est un nombre entier tel que le poids moléculaire moyen est compris entre 1 000 et 100 000 ;

et/ou un de ses sels.

2. Composition cosmétique, caractérisée en ce qu'elle contient de 0,1 à 10 % en poids d'un copolymère d'acide aminé ou d'un mélange d'au moins 2 copolymères de formule statistique :

$$-\left(A_m-\left(NH-\begin{array}{c}CH\\|\\(CH_2)_a\\|\\COOH\end{array}-CO\right)_n-B_p-\left(NH-\begin{array}{c}CH\\|\\(CH_2)_b\\|\\COOH\end{array}-CO\right)_q-\right)_z \qquad (I)$$

dans laquelle
- a et b valent chacun indépendamment 1 ou 2 ;
- A et B sont semblables ou différents et représentent un acide aminé choisi parmi : Arginine, Sérine, Proline, Glycine, Histidine, Alanine, Lysine, Ornithine, Citrulline, Acide urocanique, Asparagine ou Tyrosine ;
- m, n, p, q sont des nombres entiers tels que le rapport $\frac{n+q}{m+p}$ est compris entre 0,5 et 4 ;
- z est un nombre entier tel que le poids moléculaire est compris entre 1 000 et 100 000 ;

et/ou un de leurs sels.

3. Composition cosmétique selon la revendication 2, caractérisée en ce qu'elle contient de 0,5 à 5 % en poids du copolymère d'acides aminés ou du mélange d'au moins 2 copolymères.

4. Composition cosmétique selon l'une des revendications 2 ou 3, caractérisée en ce qu'elle contient un mélange de 2 copolymères (I).

5. Composition cosmétique selon l'une des revendications 2 ou 3, caractérisée en ce que, dans la formule statistique du copolymère (I), a = b = 2.

6. Composition cosmétique selon l'une quelconque des revendications 2, 3, 4 ou 5, caractérisée en ce que, dans la formule statistique du copolymére (I), A = B et $\frac{n+q}{m+p} = 1$.

7. Composition cosmétique selon l'une quelconque des revendications 2, 3, 5 ou 6, caractérisée en ce que le copolymère (I) est Lys-Glu (1/1).

8. Composition cosmétique selon l'une quelconque des revendications 2, 3, 5 ou 6, caractérisée en ce que le copolymère (I) est Pro-Glu (1/1).

9. Composition cosmétique selon l'une quelconque des revendications 2 à 7, caractérisée en ce qu'elle contient un mélange de 2 copolymères Pro-Glu (1/1) et Lys-Glu (1/1).

Office européen des brevets

# RAPPORT DE RECHERCHE EUROPEENNE

Numero de la demande

EP 91 40 0320

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | FR-A-2 533 222 (CENTRE NATIONAL DE LA RECHERCHE SCIENTIFIQUE)<br>* En entier *<br>--- | 1-9 | A 61 K 7/48<br>A 61 K 7/06<br>A 61 K 7/16<br>C 08 G 69/10 |
| A | EP-A-0 305 282 (UNIVERSITY OF SOUTH ALABAMA)<br>* En entier *<br>--- | 1-9 | |
| A | EP-A-0 327 411 (DELALANDE S.A.)<br>* En entier *<br>--- | 1-9 | |
| A | EP-A-0 142 231 (NITTO BOSEKI CO., LTD)<br>* En entier *<br>--- | 1-9 | |
| A | DE-A-3 612 102 (CESKOSLOVENSKA AKADEMIE VED)<br>* En entier *<br>----- | 1-9 | |
| | | | **DOMAINES TECHNIQUES RECHERCHES (Int. Cl.5)** |
| | | | A 61 K<br>C 08 G |

Le présent rapport a été établi pour toutes les revendications

| Lieu de la recherche | Date d'achèvement de la recherche | Examinateur |
|---|---|---|
| LA HAYE | 18-06-1991 | FISCHER J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)